# EUROPEAN PATENT SPECIFICATION

(11) **EP 0 502 636 B1**
(45) Date of publication and mention of the grant of the patent: **14.08.1996**
(21) Application number: 92301464.1
(22) Date of filing: 21.02.1992
(51) Int. Cl.: A61K 7/043

(54) **Nail lacquer primary film forming resin**
Primäres filmformendes Harz für Nagellack
Résine primaire formant des films pour vernis à ongles

(30) Priority: 05.03.1991 US 644737
(43) Date of publication of application: 09.09.1992
(73) Proprietor: DOW CORNING CORPORATION, Midland Michigan 48686-0994 (US)
(72) Inventor: Legrow, Gary Edward, Midland, Michigan (US); Sojka, Milan Franz, Midland, Michigan (US)
(74) Representative: Dowden, Marina

(56) References cited:
- EP-A- 0 199 325
- US-A- 3 706 697
- US-A- 4 291 136
- PATENT ABSTRACTS OF JAPAN vol. 14, no. 176 (C-707)(4119) 9 April 1990

## Description

This invention relates to a primary film forming resin for nail lacquers and nail enamel formulations and more particularly is directed to a grafted polymeric film forming resin for nail polish which will function as a replacement for nitrocellulose.

Nail lacquers and enamels typically contain several ingredients among which are a primary film former, secondary film formers, plasticizers, solvents, colorants and fillers. In the past, nitrocellulose has been the primary film former employed in the majority of nail polish formulations but it suffers from the disadvantages that it is explosive; it tends to discolor as a function of time rendering it aesthetically displeasing; and it is prone to undergo sharp changes in viscosity which make application difficult. Thus a need exists in the nail lacquer arts for an improved primary film forming material.

Unlike copolymers having a linear arrangement of sequences such as -AAABBB-and -ABABAB-, graft copolymers are polymers in which the molecules are characterized by a main backbone chain to which side chains containing different atomic constituents are attached at various points along the main chain.

For example, a graft copolymer can be represented by the structure 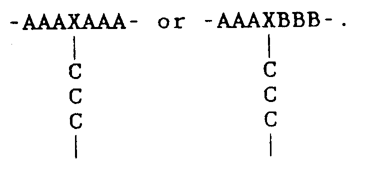 The monomer units A and B are referred to as the main chain or backbone, the sequence of C units is the side chain or graft and X is the unit in the backbone to which the graft is attached.

Nail lacquers containing graft polymers are not new. U.S. Patent No. 4683007 issued July 28, 1987 discloses a nail polish which includes nitrocellulose grafted to vinyl and acrylic monomers for the purpose of increasing the settling resistance of nitrocellulose. The graft polymers of the present invention however differ from such nitrocellulose grafts in both the main chain units and side chain units employed. In contrast to the '007 patent, the nail lacquer primary film formers of this invention are copolymers in which the main chain includes acrylic ester monomer units and methacrylic ester monomer units and wherein two differing side chains are grafted to the main chain including both carboxylic acid units and units of trialkoxysilyl functionality. While Examples 6, 7 and 10 of the '007 patent do not include nitrocellulose, the polymers formed therein contain backbones of all methacrylate units and contain no acrylate units as the polymers of the present invention. Further the polymers of the '007 patent do not include carboxyl units as grafted side chains and where they do include trialkoxysilyl units, the trialkoxysilyl units of the '007 patent are endblocking units rather than side chain grafts. Thus, there is a significant difference between the materials of the present invention and the materials of the '007 patent.

Japanese Patent No. 2-25411 dated January 26, 1990 relates to a durable coat cosmetic material including a nail enamel in which a dimethylpolysiloxane compound is copolymerized with acrylate and metlacrylate monomers. However a linear polymer instead of a grafted polymer is obtained in which the backbone includes each of acrylate, methacrylate and trialkoxysilyl units. There are also no grafted side chains and no carboxyl unit grafted side chains in distinction to the materials of the present invention. As noted above the copolymeric film formers of the present invention are graft copolymers and not copolymers having linear arrangements of sequences only.

The invention is directed to a graft copolymer comprising a main backbone chain of acrylic ester units and methacrylic ester units, the main backbone chain having grafted thereto pendant carboxyl groups and pendant trialkoxysilyl groups, wherein the carboxyl groups are grafted to only the acrylic ester units and the trialkoxysilyl groups are grafted to the remaining unsubstituted acrylic ester units.

The invention is also directed to a nail lacquer which includes a film forming resin, a plasticizer and a solvent. The improvement in accordance with the present invention relates to the utilization as the film forming resin of the nail lacquer the graft copolymer noted above.

Nail coat compositions containing the novel film forming resins of the present invention have been found to provide improved wear resistance which is a highly desirable and sought-after factor in the nail enamel market.

These and other features, objects and advantages of the herein described present invention will become more apparent when considered in light of the following detailed description.

Nail lacquer formulations exhibiting improved wear resistance are provided herein by incorporating into nail care compositions as the film forming ingredient a graft copolymer. The graft copolymer in accordance with the present invention includes a main backbone chain of acrylic ester monomer units and methacrylic ester monomer units. In addition the main backbone chain has grafted thereto side chain units of carboxyl groups and side chain units of trialkoxysilyl groups. The graft polymers of the present invention can be represented by the segment structure 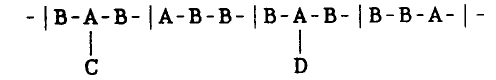 in which A represents an acrylate monomer unit, B represents a methacrylate monomer unit, C represents a carboxyl group and D represents a trialkoxysilyl group.

This random grafted copolymer contains in a typical segment of one hundred A and B units, thirty-three A units and sixty-seven B units. However, in such a one hundred unit segment there is only one C unit and one D unit. The C and D units graft to only the A units. In other words, of one hundred "mer" units of the polymer there are sixty-seven B units, thirty-one A units, one A unit that has a C unit grafted thereto and one A unit that has a D unit grafted thereto. The distribution of the "mer" units is random.

It should be apparent that the polymers of the present invention differ substantially from the materials of the prior art as represented by the aforementioned '007 patent and Japanese patent 25411. For example, the backbone of the polymer of the '007 patent is all B units and does not contain A units. There are no C units in the '007 patent and the D units of the '007 patent are attached to the ends of the backbone rather than being grafted as side chains. Since the polymer of the '007 patent contains no A units, the concept of grafting is absent. As to the Japanese patent 25411, the backbone is a combination of A, B and D units rather than A and B units. There are no C units and no grafted side chains.

The acrylic ester mononer hss the formula CH₂=CHCOOR in which R is preferably an alkyl group having from one to sixteen carbon atoms. The methacrylic ester monomer has the formula CH₂=C(CH₃)COOR' in which R' is preferably an alkyl group having one to fourteen carbon atoms. Most preferred are main backbone chain copolymers in which the acrylic ester monomer is butyl acrylate and the methacrylic ester monomer is methyl metlacrylate.

The carboxyl graft is formed by reacting the main backbone chain copolymer with acrylic acid in the presence of a free radical initiator. The trialkoxysilyl graft is formed by reacting the main backbone chain copolymer with an acrylate or a methacrylate functional silane monomer in the presence of a free radical initiator. The grafts are formed by dissolving the main backbone chain copolymer in a solvent and simultaneously reacting the copolymer with acrylic acid and the silane monomer in the presence of a free radical initiator. The silane monomer is preferably 3-methacryloxypropyltrimethoxysilane although there may also be employed methacryloxypropenyltrimethoxysilane, 3-methacryloxypropyl-tris(methoxyethoxy)silane and 3-acryloxypropyltrimethoxysilane.

While the most preferred monomers for the backbone chain copolymer are butyl acrylate and methyl methacrylate, other combinations of acrylates and methacrylates may be employed such as methyl, ethyl, propyl, pentyl, hexyl, octyl, nonyl, decyl, dodecyl, tetradecyl and hexadecyl acrylates; and ethyl, propyl, isopropyl, n-butyl, isobutyl, sec-butyl, t-butyl, n-hexyl, n-octyl, isooctyl, 2-ethylhexyl, n-decyl and tetradecyl methacrylates.

A particularly distinct advantage of the present invention is that there is produced a material which contains less than about five parts per million of residual monomer. Thus a practically mononer free product is produced by virtue of the fact that any residual monomer is grafted to the polymer. The following is a brief summary of the process for producing the monomer free product including the synthesis of a magnesium hydroxide suspension in which acrylate polymer beads are produced.

Magnesium sulfate is dissolved is water and the solution is reacted with a water solution of sodium hydroxide. This reaction is conducted at a temperature range of 20 to 50°C. while stirring the contents at a rate of 50-1000 rpm. A suspension of two to ten weight percent magnesium hydroxide in water is produced. The reaction is preferably conducted at room temperature at a stirring rate of one hundred rpm and the concentration of the final suspension is preferably five weight percent. A mixture of hydrolytically stable water insoluble organic arylate monomers together with a free radical initiator are slowly added to the magnesium hydroxide suspension at a temperature of 20 to 60°C. while stirring the contents at a rate of 200-2000 rpm. Acrylate polymer beads are produced with the ratio of polymer beads to magnesium hydroxide being a maximum of four to one. The suspension of polymer beads in water is treated with hydrochloric acid to convert the magnesium hydroxide surface layer on the beads to water soluble magnesium chloride. The beads are separated by filtration, washed and dried. The polymer beads are dissolved in a solvent such as ethyl acetate. Other solvents which may also be employed are set forth below. To the dissolved polymer is added acrylic acid, a free radical initiator and a trialkoxysilyl acrylate monomer. The concentration of acrylic acid and trialkoxysilyl acrylate monomer relative to the polymer is in the range of 0.1-10 percent by weight preferably one percent. The solution is heated for six to twelve hours at 50 to 100°C. to produce the grafted polymer. Grafting of the monomers to the polymer is complete upon a determination of the presence of no detectable monomer.

The following examples illustrate the present invention and the concepts embodied therewithin.

### Example I

Into a three neck reaction flask was placed 253.55 grams of MgSO₄(7H₂O) which had been dissolved at room temperature in 391.71 grams of deionized water. While stirring the contents of the flask there was slowly added a solution of 82.3 grams of sodium hydroxide in 678.55 grams of deionized water. The resulting suspension of magnesium hydroxide was purged with nitrogen for ten minutes. The stirring rate in the flask was adjusted to 800 RPM and in 195 grams of methyl methacrylate and 99 grams of butyl methacrylate, 5.88 grams of VAZO 64 initiator were dissolved and added slowly to the suspension. While maintaining a slow nitrogen stream flow, the suspension was heated to 60°C. upon initiation of precipitation of the methyl methacrylate-butyl acrylate polymer beads. After the elapse of two hours the system was heated to 80°C. for an additional six hours. The flask was allowed to cool to room temperature. The polymer bead suspension was neutralized to a pH of five with hydrochloric acid. Deionized water at 50°C. was used to wash the suspension and establish a neutral pH. The beads were filtered and dried in an oven at about 50°C. Using a mixer, ninety-eight grams of the beads were dissolved in 900 grams of ethyl acetate. The solution was poured into a three neck reaction flask and purged with nitrogen for ten minutes. While stirring and maintaining a slow nitrogen stream flow, there was added one gram of acrylic acid, one gram of the methacrylate functional silane monomer 3-methacryloxypropyltrimethoxysilane and two grams of VAZO 64 initiator. The grafted polymer solution in the flask was heated to 70°C. for eight hours. The grafted polymer was free of residual monomer.

### Example II

Using a mixer, 392 grams of polymer beads formed from the monomers methyl methacrylate and n-butyl methacrylate were dissolved in 600 grams of ethyl acetate. The solution was poured into a three neck reaction flask and purged with nitrogen for ten minutes. While stirring and maintaining a slow nitrogen stream flow, there was added four grams of acrylic acid, four grams of the methacrylate functional silane mononer 3-methacryloxypropyltrimethoxysilane and eight grams of VAZO 64 initiator. The grafted polymer solution in the flask was heated to 70°C. for eight hours. The grafted polymer was free of residual monomer.

### Example III

While stirring, there was blended one hundred grams of the grafted polymer solution from Example I and two hundred grams of the grafted polymer solution of Example II. The blended grafted copolymer was found to contain no detectable residual monomer.

### Example IV

Example I was repeated except that 211.28 grams of magnesium sulfate in crystal form were dissolved in 304.21 grams of deionized water. The amount of sodium hydroxide employed was 68.58 grams in 526. 98 grams of deionized water. The resulting grafted polymer contained zero parts per million of free monomer.

### Example V

Example I was repeated except that the suspension was washed three times with a fifty percent water solution of isopropyl alcohol. The polymer bead to solution ratio was one to thirty. The resulting grafted polymer contained zero parts per million of residual monomer.

### Example VI

This example is set forth for purpose of comparison. To a three neck reaction flask containing 354.5 grams of ethyl acetate and one gram of VAZO 64 initiator, there was added and dissolved sixty-five grams of methyl methacrylate, thirty-three grams of butyl acrylate, one gram of acrylic acid and one gram of the silane monomer 3-methacryloxypropyltrimethoxysilane. A nitrogen blanket was established and the system was heated to 70°C. for ten hours. The resulting polymer solution was found to contain three hundred parts per million of free monomer.

Nail enamels in accordance with the present invention can be formulated to include the grafted polymers described above as the primary film forming resin. In addition to the primary film forming resin, the nail enamels require a plasticizer, a solvent and a colorant.

The plasticizer functions to control the flexibility and the elongation of the film. Plasticizers preferably should be nonvolatile, colorless, odorless and tasteless. Some examples of appropriate plasticizers which may be employed are dibutyl phthalate, tricresyl phosphate, dibutyl phthalate, dibutyl glycolate, dioctyl phthalate, camphor, castor oil, benzyl benzoate, tributyl phosphate, butyl acetal ricenoleate, glyceryl acetal ricenoleate, butyl stearate, tributoxy ethyl phosphate, triphenyl phosphate, triethyl citrate, tributyl citrate, tributyl acetyl citrate, dibutyl tartarate, dimethoxy ethyl phthalate and diamyl phthalate.

Solvents and diluents which may be used are acetone, ethyl acetate, butyl acetate, methyl glycol acetate, methyl ethyl ketone, methyl isobutyl ketone, methyl acetate, toluene and xylene.

As a colorant there may be employed organic pigments such as The Cosmetics, Toiletries and Fragrance Association designated materials D and C Red Nos. 5-7, 10-13 and 34 and D and C Yellow Nos. 5 and 6. Cosmetic grade inorganic pigments may also be employed such as yellow and red iron oxides, brown iron oxide, iron blue, iron black, carbon black, purified titanium dioxide and bismuth oxychloride.

Nail lacquers and nail enamel formulations in accordance with the present invention contain six to thirty-five percent by weight of the primary film forming resin, five to eight percent by weight of the plasticizer, sixty to eighty percent by weight of the solvent system and about .05 to six percent by weight of colorant.

It will be apparent from the foregoing that many other variations and modifications may be made in the compounds, compositions and methods described herein without departing substantially from the essential features and concepts of the present invention. Accordingly, it should be clearly understood that the forms of the invention described herein are exemplary only and are not intended as limitations on the scope of the present invention as defined in the appended claims.

## Claims

1. A graft copolymer comprising a main backbone chain of acrylic ester units and methacrylic ester units, the main backbone chain having grafted thereto pendant carboxyl groups and pendant trialkoxysilyl groups, wherein the carboxyl groups are grafted to only the acrylic ester units and the trialkoxysilyl groups are grafted to the remaining unsubstituted acrylic ester units.

2. The copolymer of claim 1, wherein the acrylic ester monomer has the formula CH₂=CHCOOR in which R is an alkyl group having from one to sixteen carbon atoms and the methacrylic ester monomer has the formula CH₂=C(CH₃)COOR' in which R' is an alkyl group having one to fourteen carbon atoms.

3. The copolymer of claim 2, wherein the acrylic ester units are butyl acrylate and the methacrylic ester units are methyl methacrylate.

4. The copolymer of claim 1 in which the carboxyl grafts are obtainable by reacting the main backbone chain copolymer with acrylic acid in the presence of a free radical initiator.

5. The copolymer of claim 1 in which the trialkoxysilyl grafts are obtainable by reacting the main backbone chain copolymer with an acrylate or methacrylate functional silane monomer in the presence of a free radical initiator.

6. The copolymer of claim 1 in which the grafts are obtainable by dissolving the main backbone chain copolymer in a solvent and simultaneously reacting the copolymer with acrylic acid and an acrylate or methacrylate functional silane monomer in the presence of a free radical initiator.

7. The copolymer of claim 6 in which the silane monomer is 3-methacryloxypropyltrimethoxysilane.

8. A nail lacquer which comprises a film forming resin, a plasticiser and a solvent, wherein the film forming resin is a graft copolymer according to any of the preceding claims.

9. The nail lacquer of claim 8 which additionally includes a pigment.

10. A method of making the graft copolymer of claim 1 which comprises forming a main backbone chain by reacting acrylic ester units and methacrylic ester units, forming a carboxyl graft on the main backbone chain by reacting the main backbone chain with acrylic acid and forming a trialkoxysilyl graft on the main backbone chain by reacting the main backbone chain with an acrylate or methacrylate functional silane monomer, wherein the carboxyl grafts are grafted to only acrylic ester units and the trialkoxysilyl grafts are grafted to the remaining unsubstituted acrylic ester units.

## Patentansprüche

1. Pfropfcopolymer, enthaltend eine Hauptkette aus Acrylestereinheiten und Methacrylestereinheiten, wobei die Hauptkette aufgepfropfte Carboxylseitengruppen und Trialkoxysilylseitengruppen aufweist, wobei die Carboxylgruppen nur auf den Acrylestereinheiten aufgepfropft sind und die Trialkoxysilylgruppen auf den verbleibenden nichtsubstituierten Acrylestereinheiten aufgepfropft sind.

2. Copolymer nach Anspruch 1, wobei das Acrylestermonomer der Formel CH₂=CHCOOR entspricht, in der R eine Alkylgruppe mit 1-16 Kohlenstoffatomen ist und das Methacrylestermonomer der Formel CH₂=C(CH₃)COOR' entspricht, in der R' eine Alkylgruppe mit 1-14 Kohlenstoffatomen ist.

3. Copolymer nach Anspruch 2, wobei die Acrylestereinheiten Butylacrylat sind und die Methacrylestereinheiten Methylmethacrylat sind.

4. Copolymer nach Anspruch 1, bei dem die Carboxylpfropfungen erhältlich sind durch Umsetzen des Hauptkettencopolymers mit Acrylsäure in Gegenwart eines Radikalstarters.

5. Copolymer nach Anspruch 1, in dem die Trialkoxysilylpfropfungen erhältlich sind durch Umsetzen des Hauptkettencopolymers mit einem acrylat- oder methacrylatfunktionellen Silanmonomer in Gegenwart eines Radikalstarters.

6. Copolymer nach Anspruch 1, bei dem die Pfropfungen erhältlich sind durch Auflösen des Hauptkettencopolymers in einem Lösungsmittel und gleichzeitiges Umsetzen des Copolymers mit Acrylsäure und einem acrylat- oder methacrylatfunktionellen Silanmonomer in Gegenwart eines Radikalstarters.

7. Copolymer nach Anspruch 6, bei dem das Silanmonomer 3-Methacryloxypropyltrimethoxysilan ist.

8. Nagellack, der ein filmbildendes Harz, einen Weichmacher und ein Lösungsmittel enthält, wobei das filmbildende Harz ein Pfropfcopolymer nach einem der vorstehenden Ansprüche ist.

9. Nagellack nach Anspruch 8, der zusätzlich ein Pigment enthält.

10. Verfahren zur Herstellung des Pfropfcopolymers nach Anspruch 1, das Ausbilden einer Hauptkette durch Umsetzen von Acrylestereinheiten und Methacrylestereinheiten, Ausbilden einer Carboxylpfropfung auf der Hauptkette durch Umsetzen der Hauptkette mit Acrylsäure und Ausbilden einer Trialkoxysilylpfropfung auf der Hauptkette durch Umsetzen der Hauptkette mit einem acrylat- oder methacrylatfunktionellen Silanmonomer umfaßt, wobei die Carboxylpfropfungen nur auf Acrylestereinheiten aufgepfropft werden und die Trialkoxysilylpfropfungen auf den verbleibenden unsubstituierten Acrylestereinheiten aufgepfropft werden.

## Revendications

1. Un copolymère greffé comprenant une chaîne principale de squelette constituée de motifs d'ester acrylique et de motifs d'ester méthacrylique, des groupes carboxyle latéraux et des groupes trialcoxysilyles latéraux étant greffés à la chaîne principale de squelette, dans lequel les groupes carboxyle sont greffés uniquement aux motifs d'ester acrylique et les groupes trialcoxysilyles sont greffés aux motifs d'ester acrylique non substitués restants.

2. Le copolymère de la revendication 1, dans lequel le monomère du type ester acrylique répond à la formule CH₂=CHCOOR où R est un groupe alkyle ayant un à seize atomes de carbone et le monomère du type ester méthacrylique répond à la formule CH₂=C(CH₃)COOR' où R' est un groupe alkyle ayant un à quatorze atomes de carbone.

3. Le copolymère de la revendication 2, dans lequel les motifs d'ester acrylique sont formés d'acrylate de butyle et les motifs d'ester méthacrylique sont formés de méthacrylate de méthyle.

4. Le copolymère de la revendication 1, dans lequel les greffons carboxyle peuvent être obtenus en faisant réagir le copolymère formant la chaîne principale de squelette avec l'acide acrylique en présence d'un initiateur de réaction radicalaire.

5. Le copolymère de la revendication 1, dans lequel les greffons trialcoxysilyles peuvent être obtenus en faisant réagir le copolymère formant la chaîne principale de squelette avec un monomère du type silane à fonction acrylate ou méthacrylate en présence d'un initiateur de réaction radicalaire.

6. Le copolymère de la revendication 1, dans lequel les greffons peuvent être obtenus en dissolvant le copolymère formant la chaîne principale de squelette dans un solvant et en faisant réagir simultanément le copolymère avec l'acide acrylique et un monomère du type silane à fonction acrylate ou méthacrylate en présence d'un initiateur de réaction radicalaire.

7. Le copolymère de la revendication 6, dans lequel le monomère du type silane est le 3-méthacryloxypropyltriméthoxysilane.

8. Un vernis à ongles qui comprend une résine filmogène, un plastifiant et un solvant, dans lequel la résine filmogène est un copolymère greffé selon l'une quelconque des revendications précédentes.

9. Le vernis à ongles de la revendication 8, qui contient de plus un pigment.

10. Un procédé de fabrication du copolymère greffé de la revendication 1, qui consiste à former une chaîne principale de squelette en faisant réagir des motifs d'ester acrylique et des motifs d'ester méthacrylique, à former un greffon carboxyle sur la chaîne principale de squelette en faisant réagir la chaîne principale de squelette avec l'acide acrylique et à former un greffon trialcoxysilyle sur la chaîne principale de squelette en faisant réagir la chaîne principale de squelette avec un monomère du type silane à fonction acrylate ou méthacrylate, les greffons carboxyle étant greffés uniquement sur des motifs d'ester acrylique et les greffons trialcoxysilyles étant greffés sur les motifs d'ester acrylique non substitués restants.
